# NEW EUROPEAN PATENT SPECIFICATION

(11) **EP 0 168 833 B2**
(45) Date of publication and mention of the opposition decision: **12.10.1994**
(45) Mention of the grant of the patent: 26.04.1989
(21) Application number: 85108990.4
(22) Date of filing: 18.07.1985
(51) Int. Cl.: A61B 17/06, C23C 22/24

(54) **Sterile surgical needle having dark non-reflective surface**
Sterile chirurgische Nadel mit dunkler nichtreflektierender Oberfläche
Aiguille chirurgicale stérile à surface noire non réfléchissante

(30) Priority: 19.07.1984 US 632343
(43) Date of publication of application: 22.01.1986
(73) Proprietor: ETHICON INC., Somerville New Jersey 08876 (US)
(72) Inventor: Bendel, Lee, Lebanon, N.J. 08837 (US); Stoffel, Florence, Bridgewater, N.J. 08876 (US)
(74) Representative: Groening, Hans Wilhelm, Dipl.-Ing.

(56) References cited:
- EP-A- 0 052 056
- CH-A- 322 486
- FR-A- 2 287 892
- US-A- 2 192 280
- US-A- 2 865 376
- US-A- 2 960 420
- US-A- 3 210 220
- US-A- 3 437 532
- US-A- 3 899 367
- US-A- 3 915 759

## Description

The present invention relates to sterile surgical needles, and more particulary to such needles which have a uniformly dark and non-reflective surface and to a method for manufacturing such needles.

### Background of the present invention

In the past, sterile surgical needles generally have had a bright or shiny chromium or silver type of surface. It was thought this shiny surface, which was a result of polishing for the most part, was required in order to obtain desired sharpness and cutting characteristics or penetration characteristics with the needle. A drawback to these shiny surfaces is the difficulty they present in being observed by the surgeon during a surgical procedure. Cardiovascular surgeons and micro-surgeons have found it quite difficult to use needles which reflect light in surgery because of the reduced visibility of such needles. With the advent of micro-surgery there is a similar problem in lack of visibility within the surgical site of these highly reflective needles.

Methods of blackening the reflective surfaces of metal materials have been known for some time and have been attempted with surgical needles. To the best of my knowledge, no one has been able to develop a suitably dull, non-reflective needle as these blackening processes suffer from one or more disadvantages. Often these blackening processes form a coating on the needle which may flake off during use. In some instances the processes form a non-uniform non-reflective surface which causes visibility problems as well as interfering with the sharpness characteristics of the needle. In most of these blackening processes the needle becomes dull as a result of the blackening treatment.

EP-A-0 052 056 discloses a method for producing a selective absorption sheet of solar radiation wherein a stainless steel sheet is subjected to a chemical conversion treatement in an acidic oxidizing bath, which contains from 70 to 90 g/I of at least one member selected from the group consisting of sodium bichromate and potassium bichromate and from 550 to 630 g/I of sulphuric acid, at a temperature range of from 80 to boiling point of the acidic oxidizing bath (approximately 150 _{°} C), thereby forming on the stainless steel sheet a film of metal oxides or metal hydroxides, in which film the metal is dispersed with a concentration gradient from the surface towards the interior of the film. Furthermore, the temperature of chemical conversion treatment is in this known method from 105 to 1100 C.

What I have discovered is a new method for producing sterile surgical needles having a dark and non-reflective surface. The surfaces of my new needles do not flake and my new needles have excellent sharpness and penetration characteristics. Other objects and advantages of the present invention will become readily apparent from a reading of the following detailed description and drawings.

### Summary of the present invention

In accordance with the process of the present invention as exemplified in the independent claim, my new sterile surgical needle has a uniformly dark and non-reflective surface. The surface is non-flaking and the needle has penetration characteristics substantially the same as needles having shiny and polished surfaces. The penetration characteristics of the needles manufactured according to the present invention are less than 40 grams per 25.4 µm (mil) of needle diameter to cause the needle to penetrate a given material and a penetration factor of less than 1.5 grams per 25.4 _{/1}.m (mil) of needle diameter to cause the needle to pass through the material. The preferred needles manufactured according to the present invention have a matte black surface. The method of the present invention for producing my new needles is to first select a needle made from a steel containing chromium. The needle should have the desired shape or configuration and degree of sharpness. The surface of the selected needle is treated such as by electrical processes to activate the surface by eliminating chromium rich oxides. The treated needle is submersed in a solution of sulfuric acid, potassium dichromate, and water at a temperature in excess of 100_{°}C. The needle is maintained in the bath for a sufficient period of time to uniformly darken the surface of the needle and render that surface non-reflective. The needle is removed from the bath and may be rinsed with running hot water, ultrasonically rinsed at room temperature, air dried, lubricated and oven dried. It is preferred that the needles be lubricated with silicone and dried. The needle is sterilized by various well known sterilization techniques such as gas sterilization, irradiation, or the like. In most instances, a suture will be attached to the blunt end of the needle and the needle placed in a suitable package prior to sterilization.

### Brief description of the drawings

Figure 1 is a perspective view of one shape of needle of the present invention;
Figure 2 is a cross-sectional view taken along line 2-2 of Figure 1; and
Figure 3 is a box flow sheet showing the various steps in the method of the present invention.

### Detailed description of the invention

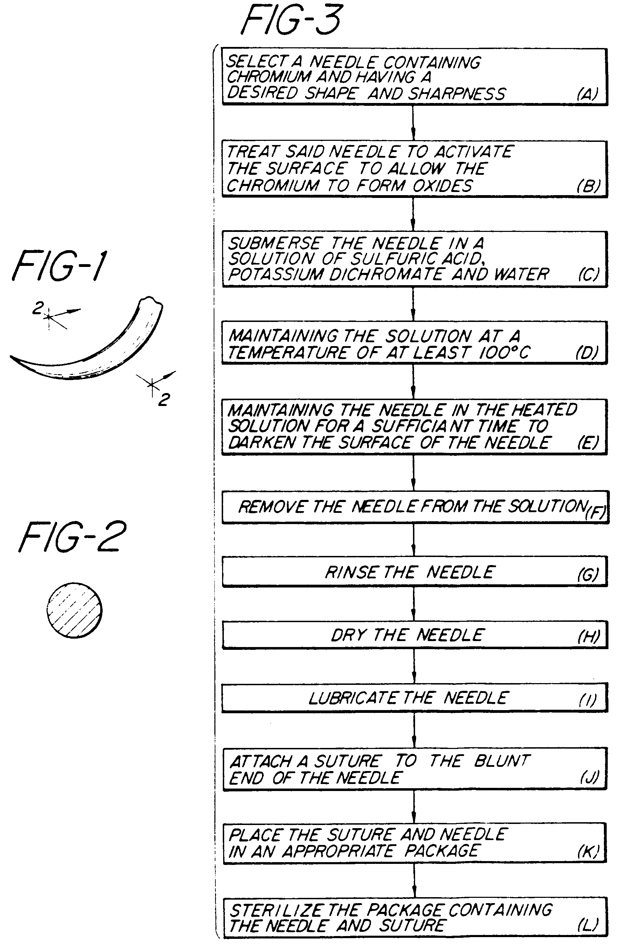

Referring to the drawings, in Figure 1 there is shown one shape of needle of the present invention. In this figure there is shown a curved needle. As shown in Figure 2 the cross-sectional shape of the needle, at least of the pointed end, is circular. The blunt end of the needle may have a suture attached thereto and in many instances the sides of the needle will be flattened to assist in grasping of the needle by a suitable needle holding instrument. The entire surface of the needle is dark and non-reflective and the surface is uniformly dark and non reflective. The surface is also non-flaking and the needle has excellent penetration characteristics. In the preferred embodiments of the needles of the present invention, the needle has a matte black finish. The needles of the present invention may be made from any of the various steels containing chromium. The preferred materials are 300 or 400 series stainless steels.

To determine the characteristics of the needle surface the needles are tested for faking characteristics. The tenacity and the non-faking characteristics of the surface are evaluated by grasping the needle with a serrated jawed needle holder such as a Codman Classic Plus needle holder. The needle holder is closed to the first locking position and released. The needle is examined under a scanning electron microscope at a magnification of approximately 500 times to determine whether any of the material has flaked. Also, the needles are examined at a magnification of 20 times to determine the intensity of the color, the edge quality and the uniformity of the colored needle. The blackened needles are passed through a chamois under a microscope to see if any of the surface of the coating comes off on the chamois. The penetration performance of the needle is determined utilizing a curved needle penetration tester. Two sensations are determined when testing the needle for penetration. The first is, the force to initially penetrate the point through a standard material and the second is to determine the drag on a needle as it passes through the appropriate material. The combination of these forces is reflected in the total force to penetrate and is measured on an Instron tester.

Figure 3 is a block flow diagram depicting the various steps which may be used to produce the needles of the present invention. First and as shown in Box A, the needle having the desired shape and a desired degree of sharpness and made from a steel containing chromium is selected. The selected needle is treated (Box B) to render the surface of the needle activated; that is, to allow the chromium on the surface of the needle to form oxides. Two preferred techniques for activating the surface of the needle are to either electroclean or electropolish the needle immediately before the blackening treatment. If the surface of the needle is not suitably activated, a thin layer of the metal is removed in the process and the cutting edges dulled and the surface roughened or channeled. The dulling and roughening greatly increases the penetration forces required with the needle and make the needle unsuitable for use in many surgical procedures where tissue trauma must be kept to a minimum. The treated needle is submersed in a solution of sulfuric acid, potassium dichromate, and water (Box C). Solutions having a specific gravity of about 1.5 to 1.6 have been found satisfactory. The solutions contain from about 60 to about 65 percent by weight of sulfuric acid and from about 7 to about 10 percent of potassium chromate with the remainder being water. The solution is heated to a temperature of at least 100_{°}C. and maintained above 100°C and below the boiling point of the solution or about 135_{°}C (Box D). The needle is maintained in the solution for a sufficient length of time to darken the needle surface (Box E). Periods of time of from about 4 minutes up to 30 minutes or even longer have been found satisfactory. Other dichromate salts, such as sodium dichromate may also be used.

The needle is removed from the solution (Box F) and the needle rinsed to remove solution residue by running hot water over the needle (Box G). The needle is preferably ultrasonically rinsed and then dried in an oven or air-dried as desired (Box H). The needle is lubricated (Box I) and an appropriate suture is attached to the blunt end of the needle (Box J). The blunt end of the needle would usually have a drilled hole or a channel with a suture swaged into the hole or channel as is well known in the art. The needle and suture is packaged in a suitable package (Box K) and the package sterilized (Box L) by cobalt radiation or ethylene oxide or other sterilization techniques as are well known in the art.

The invention will be more fully described by the following specific examples.

### Example I

A solution is prepared utilizing 180 milliliters of sulfuric acid, 50 grams of potassium dichromate and 200 milliliters of water. The solution has a specific gravity of from 1.52 to 1.55 and is maintained at a temperature of about 120 _{°} to 130 _{°} C. A curved needle having a round cross section and made from 45500 stainless steel is electro-polished by immersing the needle in a suitable polishing acid and passing a current through the needle using the needle as an anode for from 4 to 60 seconds or more. The polished needle is immersed in the solution for from about 4 to 15 minutes. The needle is removed from the hot solution and rinsed ultrasonically with water. The needle is air dried, lubricated, oven dried and the dry needle examined and tested. The needle is examined under a scanning electron microscope by grasping the needle with a serrated jawed needle holder. The needle has a good matte black surface which is non-reflective and when examined under the electron scanning microscope no faking of the black surface is detected.

The needle is tested for penetration characteristics by placing the needle in a rotating arm and pushing the needle through the penetration media following the arc of the needle. A load cell measures the force on the penetration media as the needle passes through. The data is recorded on an X-Y recorder. The needle is 25.4 µm (10 mils) in diameter and the penetration results are for initial penetration a force of 40 grams and for the drag on the needle as it passes through the media 18 grams.

### Example II

Similar needles are treated in the same bath. Needles are treated maintaining the bath at a temperature of 120 _{°} C. Needles which have had their surfaces treated with electropolishing and untreated needles are placed in the bath for from 5 to 30 minutes. In some cases, the needle has some blackening on the surface but it is not uniform and in other cases the black surface which, though it may be uniform, is shiny and reflective.

### Example III

A needle which has not been treated to activate the surface is placed in the bath described in Example I while the bath is maintained at a temperature of 120 to 130 °C. The needle is maintained in the bath for about 15 minutes. The resulting needle has a matte black surface; however, 1/10 to 2/10 of the thickness of the surface is removed and the geometry of the cutting edges are altered and the tapered point is blunted. The needle is unsuitable as a surgical needle.

### Example IV

Needles as described in conjunction with Example I are placed in an oxidizing bath. The solution used is an alkaline, oxidizing type, black oxidizing compound sold by Ethone, Inc. a subsidiary of ASARCO under the tradename Ebanol SS.52. The bath is maintained at a temperature of about 121 _{°} to 126°C. Some of the needles are pretreated by electropolishing to activate the needle surface. In all instances, the untreated or pretreated needles have uniform black surface. In some instances it is a shiny black surface. When these needles are tested for faking, the blackened surface readily flakes and the needles are unsuitable for surgical use.

### Example V

Needles as described in conjunction with Example I are treated utilizing a molten salt bath of sodium dichromate The molten bath is maintained at a temperature of 468.5 (875 _{°} F) and the needles are placed in the bath for about 30 to 45 minutes. The resultant needles are tested for surface characteristics under a scanning electro microscope. The surface has been considerably roughened and penetration studies show considerable increases in the force to penetrate. The resulting needles are not suitable for surgical use. Though the process might be optimized to produce acceptable needles the hazards associated with this process render it unsuitable for desired commercial manufacture.

Having now described the invention, it should be readily apparent that many variations and modifications may be made without departing from the scope of the present invention.

## Claims

1. A method of manufacturing sterile surgical needles having uniform dark and non-reflective surfaces, said surfaces being non-flaking, said method comprising:
(a) selecting a needle made from a steel containing chromium, said needle having a desired configuration and sharpness,
(b) treating the surface of said needle to activate the needle surface to allow the chromium to form oxides,
(c) submersing said treated needle in a solution of sulfuric acid, water and potassium dichromate or sodium dichromate,
(d) maintaining the temperature of the solution in excess of 100°C. while the needle is submersed,
(e) maintaining the needle in said heated solution for a sufficient length of time to uniformly darken the surface whereby said surface is non-reflective,
(f) removing the needle from the bath and
(g) sterilizing the needle.

2. The method according to Claim 1 wherein the needle removed from the bath is rinsed with water, dried and lubricated.

3. The method according to Claim 1 wherein the treatment comprises either electro cleaning or electro polishing the needle.

4. The method according to Claim 1 wherein the solution is maintained at a temperature of from 110_{°}C. to 130°C.

5. A method according to Claim 4 wherein the needle is maintained in the solution for a period of time from 4 to 15 minutes.

6. A method according to Claim 1 wherein the needle is treated by electro cleaning or electro polishing and the needle, when removed from the bath is rinsed with water, dried and lubricated.

7. A method according to claim 1 wherein the solution is maintained at a temperature of from about 110_{°}C. to 130_{°}C. and the needle is maintained in the solution for a period of time of at least 4 minutes.

## Patentansprüche

1. Ein Verfahren zur Herstellung steriler chirurgischer Nadeln mit gleichmäßigen dunklen und nicht-reflektierenden Oberflächen, wobei die genannten Oberflächen nicht blättern, dieses genannte Verfahren umfaßt:
(a) Auswahl einer Nadel, die aus einem Stahl hergestellt ist, der Chrom enthält, wobei die genannte Nadel eine gewünschte Gestalt und Schärfe hat,
(b) Behandeln der Oberfläche der genannten Nadel, um die Nadeloberfläche zu aktivieren, um das Ausbilden von Chromoxiden zu ermöglichen,
(c) Eintauchen der behandelten Nadel in eine Lösung aus Schwefelsäure, Wasser und Kaliumdichromat oder Natriumdichromat,
(d) Aufrechterhalten der Lösungstemperatur oberhalb 100 °C, während die Nadel eingetaucht ist,
(e) Halten der Nadel in der erhitzten Lösung für eine hinreichende Zeitdauer, um die Oberfläche gleichmäßig zu dunkeln, wodurch die Oberfläche nicht reflektierend wird,
(f) Entfernen der Nadel aus dem Bad, und
(g) Sterilisieren der Nadel.

2. Das Verfahren gemäß Anspruch 1, wobei die aus dem Bad entfernte Nadel mit Wasser gespült, getrocknet und eingefettet wird.

3. Das Verfahren gemäß Anspruch 1, bei dem die Behandlung entweder eine elektrische Reinigung oder ein elektrisches Polieren der Nadel umfaßt.

4. Das Verfahren gemäß Anspruch 1, bei dem die Lösung bei einer Temperatur von 110 °C bis 130 _{°} C gehalten wird.

5. Ein Verfahren gemäß Anspruch 4, bei dem die Nadel in der Lösung für eine Zeitdauer von 4 bis 15 Minuten gehalten wird.

6. Ein Verfahren gemäß Anspruch 1, bei dem die Nadel durch elektrisches Reinigen oder elektrisches Polieren behandelt wird und die Nadel, wenn sie aus dem Bad entfernt ist, mit Wasser gespült, getrocknet und gefettet wird.

7. Ein Verfahren gemäß Anspruch 1, bei dem die Lösung bei einer Temperatur von etwa 110 _{°} C bis 130 °C und die Nadel in der Lösung für eine Zeitdauer von mindestens 4 Minuten gehalten werden.

## Revendications

1. Procédé de préparation d'aiguilles chirurgica- les stériles ayant des surfaces uniformément noires et non réfléchissantes, lesdites surfaces étant non écaillantes, ledit procédé comprenant :
(a) le choix d'une aiguille faite d'un acier contenant du chrome, ladite aiguille ayant une configuration et une acuité désirées,
(b) le traitement de la surface de ladite l'aiguille pour activer la surface de l'aiguille afin de permettre au chrome de former des oxydes,
(c ) l'immersion de ladite aiguille traitée dans une solution d'acide sulfurique, de dichromate de potassium ou de dichromate de sodium et d'eau,
(d) le maintien de la température de la solution au-dessus de 100 °C tandis qu'on plonge l'aiguille,
(e) le maintien de l'aiguille dans ladite solution chauffée pendant une durée suffisante pour noircir uniformément la surface, grâce à quoi ladite surface est non réfléchissante,
(f) l'enlèvement de l'aiguille du bain et
(g) la stérilisation de l'aiguille.

2. Procédé selon la revendication 1 dans lequel on rince l'aiguille enlevée du bain avec de l'eau, on la sèche et on la lubrifie.

3. Procédé selon la revendication 1, dans lequel le traitement comprend un électronettoyage ou un électropolissage de l'aiguille.

4. Procédé selon la revendication 1, dans lequel la solution est maintenue à une température de 110_{°} à 130°C.

5. Procédé selon la revendication 4, dans lequel on maintient l'aiguille dans la solution pendant une durée allant de 4 à 15 minutes.

6. Procédé selon la revendication 1 par lequel on traite l'aiguille par électronettoyage ou électropolissage et on rince l'aiguille, une fois enlevée du bain avec de l'eau, on la sèche et on la lubrifie.

7. Procédé selon la revendication 1, dans lequel on maintient la solution à une température d'environ 110° à à 130°C et on maintient l'aiguille dans la solution pendant une durée d'au moins 4 minutes.
